# EUROPÄISCHE PATENTSCHRIFT

(11) **EP 1 670 539 B1**
(45) Veröffentlichungstag und Bekanntmachung des Hinweises auf die Patenterteilung: **03.01.2007**
(21) Anmeldenummer: 05733261.1
(22) Anmeldetag: 23.04.2005
(51) Int. Cl.: A61M 39/22, F16K 31/50, F16K 3/24

(54) **MEDIZINTECHNISCHE VENTILEINRICHTUNG FÜR SAUG- UND/ODER SPÜLLEITUNGEN MEDIZINISCHER INSTRUMENTE**
MEDICAL VALVE MECHANISM FOR SUCTION AND/OR FLUSHING TUBES OF MEDICAL INSTRUMENTS
DISPOSITIF DE SOUPAPES MEDICAL POUR CONDUITS D'ASPIRATION ET/OU DE RINÇAGE D'APPAREILS MEDICAUX

(30) Priorität: 24.04.2004 DE 102004020071
(43) Veröffentlichungstag der Anmeldung: 21.06.2006
(73) Patentinhaber: Karl Storz GmbH & Co. KG, 78532 Tuttlingen (DE)
(72) Erfinder: EISENKOLB, Peter, 78532 Tuttlingen (DE); EFINGER, Andreas, 78604 Rietheim (DE)
(74) Vertreter: Hofmeister, Frank
(86) Internationale Anmeldenummer: PCT/EP2005/004388
(87) Internationale Veröffentlichungsnummer: WO 2005/102443

(56) Entgegenhaltungen:
- EP-A- 0 463 363
- EP-A- 1 403 574
- DE-A1- 19 604 122
- US-A- 4 794 913

## Beschreibung

Die Erfindung betrifft eine medizintechnische Ventileinrichtung für Saug- und/oder Spülleitungen medizinischer Instrumente, insbesondere Instrumente für die endoskopische Chirurgie, mit einem mit einer Durchgangsbohrung versehenen Ventilgehäuse sowie einem verstellbar im Ventilgehäuse gelagerten Ventilkörper, über den die Durchgangsbohrung verschließbar und wieder freigebbar ist, wobei der Ventilkörper als über einen Stellantrieb im wesentlichen rechtwinklig zur Durchgangsbohrung verlagerbarer Stempel ausgebildet ist, wobei eine Rotationsbewegung des Stellantriebs in eine Axialbewegung des Stempels umwandelbar ist.

Insbesondere bei der endoskopischen Chirurgie ist es notwendig das Operationsgebiet fortlaufend zu spülen und die Spülflüssigkeit und Blut aus dem Operationsgebiet abzusaugen, um im Operationsgebiet für gute Licht- und Sichtverhältnisse zu sorgen und ein übersichtliches Operieren zu ermöglichen. Das Saugen und Spülen erfolgt in der Regel über separate Saug-Spül-Einheiten mit angeschlossenen Saug- und/oder Spülleitungen. Diese Saug- und/oder Spülleitungen können entweder über separate Zugänge dem Operationsgebiet zugeführt werden oder aber in ein Operationswerkzeug integriert sein.

Um die Saug- und/oder Spülfunktion gezielt und dosiert einsetzen zu können, sind die Saug- und/oder Spülleitungen mit medizintechnischen Ventileinrichtungen versehen, über die die Saug- und/oder Spülleitungen geöffnet und wieder verschlossen werden können.

Aus der US 4 794 913 A ist eine medizintechnische Ventileinrichtung für Saug- und/oder Spülleitungen medizinischer Instrumente, insbesondere Instrumente für die endoskopische Chirurgie, bekannt, die mit einem verschwenkbaren Handhebel versehen ist. Diese bekannte Ventileinrichtung dient dazu, eine von axial nach radial führenden Strömungskanal zu verschließen. Die Umlenkung der Strömung bei dieser Konstruktion bewirkt jedoch starke Turbulenzen, wodurch eine reproduzierbare Einstellung des zu regulierenden Volumenstroms behindert wird.

Neben weiteren aus der Praxis bekannten Hähnen mit einem durchbohrten Küken ist es aus der DE 691 22 314 T2 bekannt, die Ventileinrichtungen als Schiebeventile auszubilden. Diese bekannten Schiebeventile weisen einen verschiebbar im Ventilgehäuse gelagerten Ventilkörper auf, wobei der Ventilkörper mit einer Durchgangsbohrung versehen ist, die bei gedrücktem Ventil mit der Durchgangsbohrung im Ventilgehäuse fluchtet und so die Offenstellung der Ventileinrichtung bildet.

Nachteilig bei den als Hähnen und Schiebeventilen ausgebildeten Ventileinrichtungen ist, dass die Dosierung des Volumenstroms nur schlecht dosierbar und reproduzierbar ist. Darüber hinaus verursachen die bekannten Ventileinrichtungen starke Turbulenzen im zu dosierenden Volumenstrom.

Davon ausgehend liegt der Erfindung die **Aufgabe** zugrunde, eine medizintechnische Ventileinrichtung der eingangs genannten Art zu schaffen, die eine reproduzierbare Einstellung des zu regulierenden Volumenstroms bei gleichzeitiger Verminderung von Turbulenzen ermöglicht.

Die **Lösung** dieser Aufgabenstellung ist erfindungsgemäß dadurch gekennzeichnet, dass der Stellantrieb als um die Längsachse des Stempels verschwenkbarer Handhebel ausgebildet ist und zur Überführung des Stempels von der die Durchgangsbohrung freigebenden in die die Durchgangsbohrung verschließenden Stellung um 90° bezüglich der Längsachse des Stempels verstellbar ist.

Durch die erfindungsgemäße Ausbildung des Stellantriebs kann ein schnelles Öffnen und Schließen der Ventileinrichtung dadurch bewirkt werden, dass es einer nur geringen Betätigung des Stellantriebs bedarf, um den Stempel in die jeweilige Endlage zu überführen.

Durch die Ausbildung des Ventilkörpers als in die Durchgangsbohrung des Ventilgehäuses verfahrbarer Stempel ist es auf einfache Weise möglich, den Volumenstrom reproduzierbar zu drosseln. Da der Stempel keine Durchgangsbohrung aufweist, bewirkt das Verlagern des Stempels in die Durchgangsbohrung des Ventilgehäuses hinein bzw. aus dieser heraus ein direktes Verschließen bzw. Öffnen der angeschlossenen Spül- und/oder Saugkanäle. Darüber hinaus verursacht der erfindungsgemäße Stempel weniger Turbulenzen im zu dosierenden Volumenstrom als die aus dem Stand der Technik bekannten Ventileinrichtungen.

Gemäß einer ersten praktischen Ausführungsform der Erfindung sind der Stellantrieb und der Stempel über ein Gewinde miteinander gekoppelt, das die Umwandlung der Rotationsbewegung des Stellantriebs in die Axialbewegung des Stempels bewirkt.

Gemäß einer zweiten praktischen Ausführungsform der Erfindung sind der Stellantrieb und der Stempel über ein Getriebe miteinander gekoppelt, wobei bei dieser Ausgestaltungsform der Stellantrieb vorzugsweise als senkrecht zur Längsachse des Stempels verschwenkbarer Handhebel ausgebildet ist.

Um den Volumenstrom mittels der Ventileinrichtung einfach und zuverlässig reproduzierbar dosieren zu können, wird mit der Erfindung vorgeschlagen, dass am Ventilgehäuse und/oder am Stellantrieb eine Skalierung angeordnet ist, der die Lage des Stempels zur Durchgangsbohrung entnehmbar ist. Zusätzlich oder alternativ zu der Skalierung kann der Stellantrieb in vorgegebenen Rastschritten verstellbar ausgebildet sein.

Weiterhin wird mit der Erfindung vorgeschlagen, die Steigung des die Rotationsbewegung des Stellantriebs in die Axialbewegung des Stempels umwandelnden Gewindes derart auszubilden, dass der Drehwinkel des Stellantriebs proportional zur Sperrung bzw. Freigabe der Durchgangsbohrung, das heißt proportional zur Durchflussmenge ist, wobei das Gewinde vorteilhafterweise selbsthemmend wirkt, um eine Beeinflussung der Ventilstellung durch das durchfließende Medium zu verhindern.

Schließlich wird mit der Erfindung vorgeschlagen, dass die Bewegung des Stempels über ein Sperrelement lenkbar ist, um sicherzustellen, dass die Drehbewegung des Stellantriebs nicht zu einer Verdrehung des Stempels führt, sondern dessen Axialbewegung garantiert. Vorteilhafterweise ist dieses Sperrelement als in das Ventilgehäuse einsetzbarer Stift ausgebildet, der eine Drehung des Stempels sperrend in eine Längsnut im Stempel hineinragt.

Weitere Merkmale und Vorteile der Erfindung ergeben sich anhand der zugehörigen Zeichnung, in der ein Ausführungsbeispiel einer erfindungsgemäßen medizintechnischen Ventileinrichtung für Saug- und/oder Spülleitungen medizinischer Instrumente nur beispielhaft dargestellt ist. In der Zeichnung zeigt:
- Fig. 1: eine Seitenansicht einer erfindungsgemäßen medizintechnischen Ventileinrichtung im geöffneten Zustand;
- Fig. 2: einen Schnitt entlang der Schnittlinie II-II gemäß Fig. 1;
- Fig. 3: eine Seitenansicht gemäß Fig. 1, jedoch die Ventileinrichtung im teilweise geschlossenen Zustand darstellend,
- Fig. 4: einen Schnitt entlang der Schnittlinie IV-IV gemäß Fig. 3;
- Fig. 5: eine gegenüber den Ansichten gemäß Fig.1 und Fig. 3 um 90° gedrehte Seitenansicht der erfindungsgemäßen medizintechnischen Ventileinrichtung und
- Fig. 6: einen Schnitt entlang der Schnittlinie VI-VI gemäß Fig. 5.

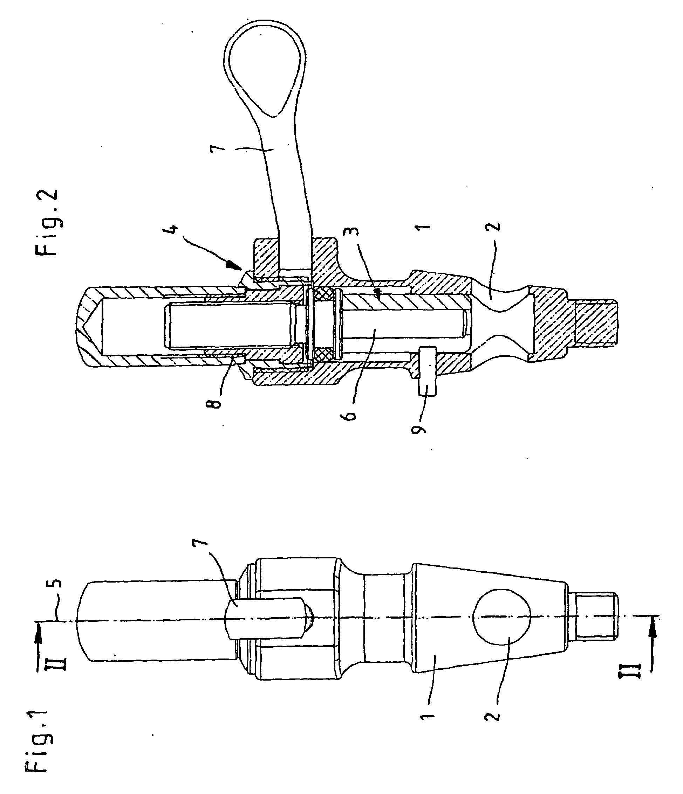

Die in den Abbildungen Fig. 1 bis Fig. 6 dargestellte medizintechnische Ventileinrichtung besteht im wesentlichen aus einem Ventilgehäuse 1 mit einer Durchgangsbohrung 2, an die nicht dargestellte Saug- und/oder Spülleitungen medizinischer Instrumente anschließbar sind, sowie einem in dem Ventilgehäuse 1 verstellbar gelagerten Ventilkörper 3.

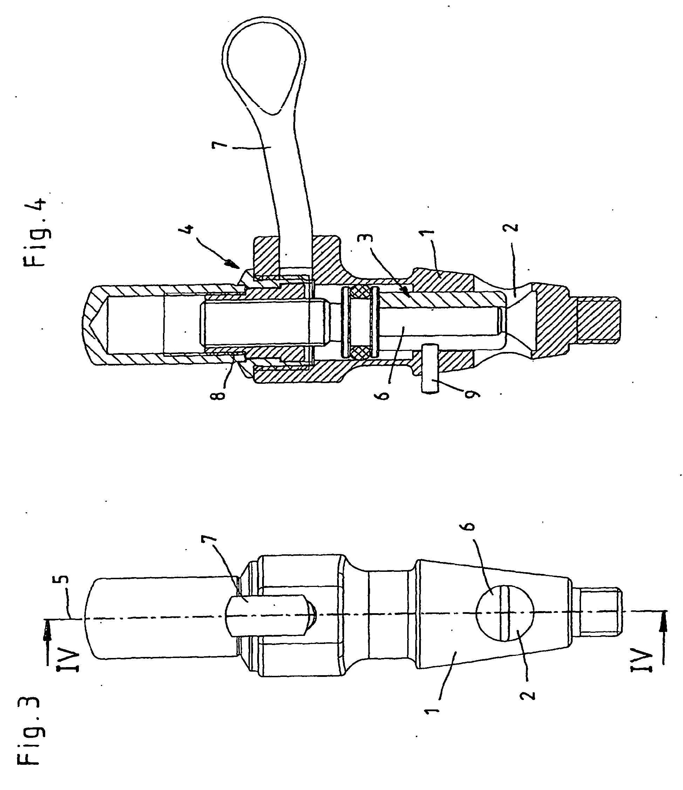

Der Ventilkörper 3 ist, wie aus den Abbildungen Fig. 1 bis 4 ersichtlich, über einen Stellantrieb 4 in Richtung der Längsachse 5 des Ventilgehäuses 1 zwischen einer die Durchgangsbohrung 2 freigebenden Offenstellung (Fig. 1 und 2) und einer die Durchgangsbohrung verschließenden Schließstellung verlagerbar im Ventilgehäuse 1 gelagert. Fig. 3 und 4 zeigen den Ventilkörper 3 in einer halb geöffneten bzw. halb geschlossenen Zwischenstellung.

Wie aus den Abbildungen ersichtlich, ist der Ventilkörper 3 als geschlossener Stempel 6 ausgebildet, der keine eigene Durchgangsbohrung aufweist, so dass jede Verlagerung des Stempels 6 über den Stellantrieb 4 ein direktes Schließen oder Öffnen der Durchgangsbohrung 2 und somit auch ein direktes Drosseln oder Verstärken des durch die Durchgangsbohrung 2 hindurchtretenden Volumenstroms bedeutet.

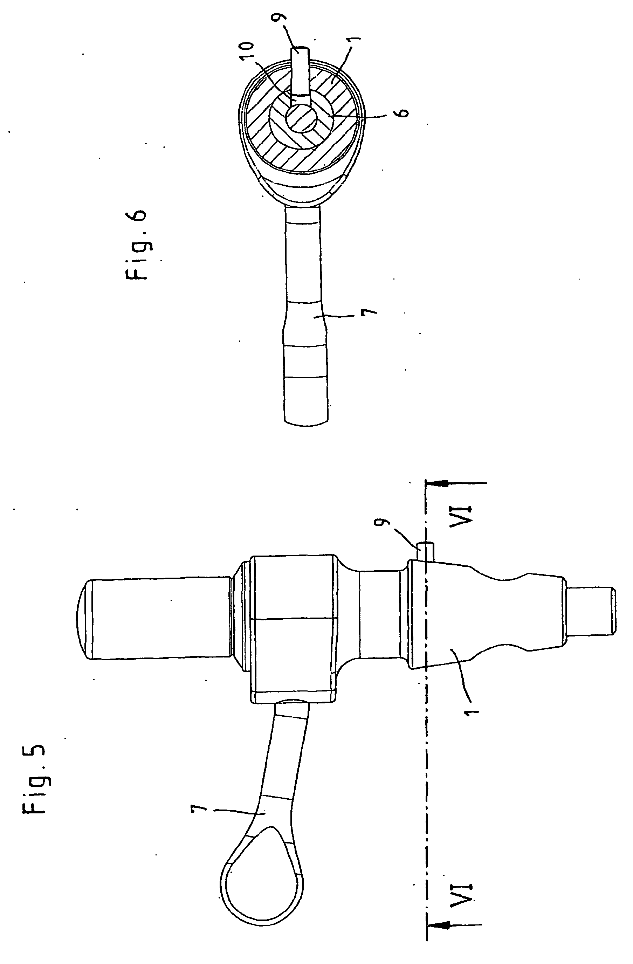

Der Stellantrieb 4 zum Betätigen des Stempels 6 ist als um die Längsachse 5 des Stempels 6 verschwenkbarer Handhebel 7 ausgebildet, der über ein Gewinde 8 so mit dem Stempel 6 gekoppelt ist, dass dieses die Rotationsbewegung des Handhebels 7 in die Axialbewegung des Stempels 6 überführt. Damit das Verschwenken des Handhebels 7 nicht zu einem Verdrehen des Stempels 6, sondern ausschließlich zum Auf- oder Abbewegen des Stempels 6 führt, ist ein als Stift ausgebildetes Sperrelement 9 vorgesehen. Wie insbesondere aus Fig. 5 und 6 ersichtlich, ragt das radial in das Ventilgehäuse 1 eingesetzte Sperrelement 9 bis in eine Längsnut 10 des Stempels 6 und verhindert so ein Verdrehen des Stempels 6.

Da der als Stempel 6 ausgebildete Ventilkörper 3 im Gegensatz zu den aus den Ventilkörpern bekannten Ventileinrichtungen selbst keine Durchgangsbohrung aufweist, steht jede Verlagerung des Ventilkörpers in einem direkten Verhältnis zur Drosselung oder Freigabe des zu dosierenden Volumenstroms, so dass diese Ventileinrichtung eine reproduzierbare und gut dosierbare Regelung des durch die angeschlossenen Saug- und/oder Spülleitungen strömenden Volumenstroms ermöglicht.

Die reproduzierbare Dosierbarkeit kann noch dadurch verbessert werden, dass am Ventilgehäuse 1 und/oder am Stellantrieb 4 eine Skalierung angeordnet ist, der die Lage des Stempels 6 zur Durchgangsbohrung 2 entnehmbar ist. Ebenso ist es möglich zusätzlich oder alternativ den Stellantrieb 4 so auszugestalten, dass er in vorgegebenen Rastschritten verstellbar ist.

Weiterhin besteht die Möglichkeit, die Steigung des die Rotationsbewegung des Stellantriebs 4 in die Axialbewegung des Stempels 6 umwandelnden Gewindes 8 derart auszubilden, dass der Drehwinkel des Stellantriebs 4 proportional zur Sperrung bzw. Freigabe der Durchgangsbohrung 2, das heißt proportional zur Durchflussmenge ist, wobei das Gewinde 8 vorteilhafterweise selbsthemmend wirkt, um eine Beeinflussung der Ventilstellung durch das durchfließende Medium zu verhindern.

Ein weiterer Vorteil der dargestellten medizintechnischen Ventileinrichtung besteht darin, dass der solchermaßen ausgebildete Ventilkörper 3 beim Öffnen und Schließen der Durchgangsbohrung 2 weniger und/oder besser kontrollierbare Turbulenzen im Volumenstrom erzeugt, wodurch wiederum die Dosierbarkeit erleichtert wird.

### Bezugszeichenliste

- 1: Ventilgehäuse
- 2: Durchgangsbohrung
- 3: Ventilkörper
- 4: Stellantrieb
- 5: Längsachse
- 6: Stempel
- 7: Handhebel
- 8: Gewinde
- 9: Sperrelement
- 10: Längsnut

## Patentansprüche

1. Medizintechnische Ventileinrichtung für Saug- und/oder Spülleitungen medizinischer Instrumente, insbesondere Instrumente für die endoskopische Chirurgie, mit einem mit einer Durchgangsbohrung (2) versehenen Ventilgehäuse (1) sowie einem verstellbar im Ventilgehäuse (1) gelagerten Ventilkörper (3), über den die Durchgangsbohrung (2) verschließbar und wieder freigebbar ist, wobei der Ventilkörper (3) als über einen Stellantrieb (4) im wesentlichen rechtwinklig zur Durchgangsbohrung (2) verlagerbarer Stempel (6) ausgebildet ist, wobei eine Rotationsbewegung des Stellantriebs (4) in eine Axialbewegung des Stempels (6) umwandelbar ist,
**dadurch gekennzeichnet,**
**dass** der Stellantrieb (4) als um die Längsachse (5) des Stempels (6) verschwenkbarer Handhebel (7) ausgebildet ist und zur Überführung des Stempels (6) von der die Durchgangsbohrung (2) freigebenden in die die Durchgangsbohrung (2) verschließenden Stellung um 90° bezüglich der Längsachse (5) des Stempels (6) verstellbar ist.

2. Medizintechnische Ventileinrichtung nach Anspruch 1, **dadurch gekennzeichnet, dass** der Stellantrieb (4) und der Stempel (6) über ein Gewinde (8) miteinander gekoppelt sind.

3. Medizintechnische Ventileinrichtung nach Anspruch 1, **dadurch gekennzeichnet, dass** der Stellantrieb (4) und der Stempel (6) über ein Getriebe miteinander gekoppelt sind.

4. Medizintechnische Ventileinrichtung nach Anspruch 3, **dadurch gekennzeichnet, dass** der Stellantrieb (4) als senkrecht zur Längsachse (5) des Stempels (6) verschwenkbarer Handhebel (7) ausgebildet ist.

5. Medizintechnische Ventileinrichtung nach mindestens einem der Ansprüche 1 bis 4, **dadurch gekennzeichnet, dass** am Ventilgehäuse (1) und/oder am Stellantrieb (4) eine Skalierung angeordnet ist, der die Lage des Stempels (6) zur Durchgangsbohrung (2) entnehmbar ist.

6. Medizintechnische Ventileinrichtung nach mindestens einem der Ansprüche 1 bis 5, **dadurch gekennzeichnet, dass** der Stellantrieb (4) in vorgegebenen Rastschritten verstellbar ist.

7. Medizintechnische Ventileinrichtung nach Anspruch 2, **dadurch gekennzeichnet, dass** die Steigung des Gewindes (8) derart ausgebildet ist, dass der Drehwinkel des Stellantriebs (4) proportional zur Lage des Stempels (6) zur Durchgangsbohrung (2) ist.

8. Medizintechnische Ventileinrichtung nach Anspruch 7, **dadurch gekennzeichnet, dass** das Gewinde (8) selbsthemmend ausgebildet ist.

9. Medizintechnische Ventileinrichtung nach mindestens einem der Ansprüche 1 bis 8, **dadurch gekennzeichnet, dass** die Bewegung des Stempels (6) über ein Sperrelement (9) lenkbar ist.

10. Medizintechnische Ventileinrichtung nach Anspruch 9, **dadurch gekennzeichnet, dass** das Sperrelement (9) als in das Ventilgehäuse (1) einsetzbarer Stift ausgebildet ist.

11. Medizintechnische Ventileinrichtung nach Anspruch 9 oder 10, **dadurch gekennzeichnet, dass** das im Stempel (6) eine Längsnut (10) für das Sperrelement (9) ausgebildet ist.

12. Medizintechnische Ventileinrichtung nach mindestens einem der Ansprüche 1 bis 11, **dadurch gekennzeichnet, dass** der Verschwenkwinkel des Stellantriebs (4) über einen Anschlag begrenzbar ist.

## Claims

1. Medical valve mechanism for suction and/or flushing tubes of medical instruments, in particular of instruments for endoscopic surgery, comprising a valve housing (1) provided with a continuous bore (2), and a valve body (3) which is mounted displaceably in the valve housing (1) and via which the continuous bore (2) can be closed and then opened again, the valve body (3) being designed as a plunger (6) which can be moved substantially perpendicular to the continuous bore (2) via an actuating drive (4), and a rotation movement of the actuating drive (4) being able to be converted into an axial movement of the plunger (6), **characterized in that** the actuating drive (4) is designed as a hand lever (7), pivotable about the longitudinal axis (5) of the plunger (6), and can be moved through 90° relative to the longitudinal axis (5) of the plunger (6) in order to bring the plunger (6) from the position opening the continuous bore (2) to the position closing the continuous bore (2).

2. Medical valve mechanism according to Claim 1, **characterized in that** the actuating drive (4) and the plunger (6) are coupled to one another via a thread (8).

3. Medical valve mechanism according to Claim 1, **characterized in that** the actuating drive (4) and the plunger (6) are coupled to one another via a gear.

4. Medical valve mechanism according to Claim 3, **characterized in that** the actuating drive (4) is designed as a hand lever (7) that can be pivoted perpendicular to the longitudinal axis (5) of the plunger (6).

5. Medical valve mechanism according to at least one of Claims 1 to 4, **characterized in that** a scale is arranged on the valve housing (1) and/or on the actuating drive (4), and the position of the plunger (6) relative to the continuous bore (2) can be taken from this scale.

6. Medical valve mechanism according to at least one of Claims 1 to 5, **characterized in that** the actuating drive (4) can be moved in predetermined latching steps.

7. Medical valve mechanism according to Claim 2, **characterized in that** the pitch of the thread (8) is configured such that the rotation angle of the actuating drive (4) is proportional to the position of the plunger (6) relative to the continuous bore (2).

8. Medical valve mechanism according to Claim 7, **characterized in that** the thread (8) is designed to be self-locking.

9. Medical valve mechanism according to at least one of Claims 1 to 8, **characterized in that** the movement of the plunger (6) can be controlled via a blocking element (9).

10. Medical valve mechanism according to Claim 9, **characterized in that** the blocking element (9) is designed as a pin that can be inserted into the valve housing (1).

11. Medical valve mechanism according to Claim 9 or 10, **characterized in that** a longitudinal groove (10) for the blocking element (9) is formed in the plunger (6).

12. Medical valve mechanism according to at least one of Claims 1 to 11, **characterized in that** the pivot angle of the actuating drive (4) can be limited by an abutment.

## Revendications

1. Dispositif à soupape à usage médical pour conduits d'aspiration et/ou de rinçage d'instruments médicaux, en particulier d'instruments destinés à la chirurgie endoscopique, comportant un boîtier de soupape (1) pourvu d'un perçage traversant (2) ainsi que d'un corps de soupape (3) monté de façon réglable dans le boîtier de soupape (1) et via lequel le perçage traversant (2) est susceptible d'être fermé et de nouveau libéré, le corps de soupape (3) étant réalisé sous la forme d'un poussoir (6) déplaçable sensiblement à angle droit par rapport au perçage traversant (2) au moyen d'un entraînement de positionnement (4), un mouvement de rotation de l'entraînement de positionnement (4) pouvant être transformé en un mouvement axial du poussoir (6),
**caractérisé en ce que** l'entraînement de positionnement (4) est réalisé sous la forme d'un levier à main (7) susceptible de pivoter autour de l'axe longitudinal (5) du poussoir (6), et pour transférer le poussoir (6) depuis la position libérant le perçage traversant (2) jusque dans la position refermant le perçage traversant (2), ledit levier est déplaçable de 90° par rapport à l'axe longitudinal (5) du poussoir (6).

2. Dispositif à soupape à usage médical selon la revendication 1, **caractérisé en ce que** l'entraînement de positionnement (4) et le poussoir (6) sont couplés l'un à l'autre via un pas de vis (8).

3. Dispositif à soupape à usage médical selon la revendication 1, **caractérisé en ce que** l'entraînement de positionnement (4) et le poussoir (6) sont couplés l'un à l'autre via un mécanisme de transmission.

4. Dispositif à soupape à usage médical selon la revendication 3, **caractérisé en ce que** l'entraînement de positionnement (4) est réalisé sous la forme d'un levier à main (7) susceptible de pivoter perpendiculairement à l'axe longitudinal (5) du poussoir (6).

5. Dispositif à soupape à usage médical selon l'une des revendications 1 à 4, **caractérisé en ce qu'**une échelle est agencée sur le boîtier de soupape (1) et/ou sur l'entraînement de positionnement (4), sur laquelle on peut lire la position du poussoir (6) par rapport au perçage traversant (2).

6. Dispositif à soupape à usage médical selon l'une des revendications 1 à 5, **caractérisé en ce que** l'entraînement de positionnement (4) est déplaçable par pas de cran prédéterminées.

7. Dispositif à soupape à usage médical selon la revendication 2, **caractérisé en ce que** le pas du pas de vis (8) est réalisé de telle sorte que l'angle de rotation de l'entraînement de positionnement (4) est proportionnel à la position du poussoir (6) par rapport au perçage traversant (2).

8. Dispositif à soupape à usage médical selon la revendication 7, **caractérisé en ce que** le pas de vis (8) est réalisé de façon autobloquante.

9. Dispositif à soupape à usage médical selon l'une des revendications 1 à 8, **caractérisé en ce que** le mouvement du poussoir (6) peut être commandé au moyen d'un élément d'arrêt (9).

10. Dispositif à soupape à usage médical selon la revendication 9, **caractérisé en ce que** l'élément d'arrêt (9) est réalisé sous la forme d'une tige susceptible d'être mise en place dans le boîtier de soupape (1).

11. Dispositif à soupape à usage médical selon la revendication 9 ou 10, **caractérisé en ce que** dans le poussoir (6) est ménagée une gorge longitudinale (10) pour l'élément d'arrêt (9).

12. Dispositif à soupape à usage médical selon l'une des revendications 1 à 11, **caractérisé en ce que** l'angle de pivotement de l'entraînement de positionnement (4) est susceptible d'être limité par une butée.
